# EUROPÄISCHE PATENTSCHRIFT

(11) **EP 1 075 476 B1**
(45) Veröffentlichungstag und Bekanntmachung des Hinweises auf die Patenterteilung: **04.06.2003**
(21) Anmeldenummer: 99920814.3
(22) Anmeldetag: 29.04.1999
(51) Int. Cl.: C07D 413/00, A61K 31/42, A61K 31/415, C07D 405/04, C07D 413/04, C07D 413/14

(54) **BENZIMIDAZOLE UND OXAZOLE**
NOVEL BENZIMIDAZOLES AND BENZOXAZOLES
NOUVEAUX BENZIMIDAZOLES ET BENZOXAZOLES

(30) Priorität: 05.05.1998 EP 98108125
(43) Veröffentlichungstag der Anmeldung: 14.02.2001
(73) Patentinhaber: ALTANA Pharma AG, 78467 Konstanz (DE)
(72) Erfinder: MARTIN, Thomas, D-78462 Konstanz (DE); ULRICH, Wolf-Rüdiger, D-78464 Konstanz (DE); AMSCHLER, Hermann, . (DE); SCHMIDT,Beate, D-78476 Allensbach (DE); FLOCKERZI, Dieter, D-78476 Allensbach (DE); HATZELMANN, Armin, D-78467 Konstanz (DE); BOSS, Hildegard, D-78476 Allensbach (DE); BEUME, Rolf, D-78465 Konstanz (DE); KILIAN, Ulrich, D-78479 Reichenau (DE); KLEY, Hans-Peter, D-78476 Allensbach (DE); BÄR, Thomas, D-78479 Reichenau (DE)
(86) Internationale Anmeldenummer: EP9902900
(87) Internationale Veröffentlichungsnummer: WO99057115

(56) Entgegenhaltungen:
- WO-A-96/03399
- WO-A-96/11917

## Beschreibung

### Anwendung der Erfindung

Die Erfindung betrifft neue Benzimidazole sowie -oxazole, die in der pharmazeutischen Industrie zur Herstellung von Medikamenten verwendet werden.

### Bekannter technischer Hintergrund

In der internationalen Anmeldung WO94/12461 werden unter anderem 2-(substituiertes Phenyl)-benzimidazole als selektive Inhibitoren der Phosphodiesterase des Typs 4 beschrieben. In der internationalen Anmeldung WO96/11917 werden 2-(substituiertes Phenyl)-benzoxazole als selektive PDE4 Inhibitoren offenbart.

### Beschreibung der Erfindung

Es wurde nun gefunden, daß die nachfolgend näher beschriebenen neuen Verbindungen der allgemeinen Formel l überraschende und besonders vorteilhafte Eigenschaften besitzen.

Gegenstand der Erfindung sind somit Verbindungen der Formel l, 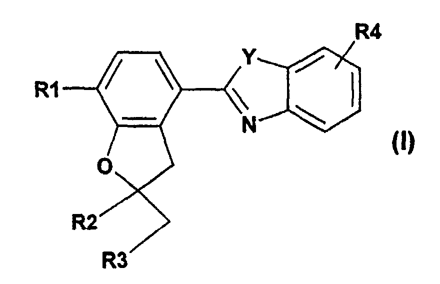 worin
- Y: O (Sauerstoff) oder NH bedeutet,
- R1: 1-4C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
- R2: Wasserstoff oder 1-4C-Alkyl und
- R3: Wasserstoff oder 1-4C-Alkyl bedeuten,
oder worin
- R2 und R3: gemeinsam und unter Einschluß der beiden Kohlenstoffatome, an die sie gebunden sind, einen spiro-verknüpften 5-, 6- oder 7-gliedrigen, gewünschtenfalls durch ein Sauerstoffatom unterbrochenen Kohlenwasserstoffring darstellen,
- R4: C(O)R5 C(O)R6, -CₙH₂ₙ-C(O)R5 oder -CₙH₂ₙ-C(O)R6 bedeutet, wobei
- R5: Hydroxy, 1-7C-Alkoxy, 3-7C-Cycloalkoxy, 3-7C-Cycloalkylmethoxy, H₂N-CₘH₂ₘ-O- oder R51(R52)N-CₚH₂ₚ-O- bedeutet, wobei
- R51: Wasserstoff, 1-7C-Alkyl, 3-7C-Cycloalkyl oder 3-7C-Cycloalkylmethyl bedeutet und
- R52: 1-7C-Alkyl, 3-7C-Cycloalkyl oder 3-7C-Cycloalkylmethyl bedeutet, oder wobei
- R51 und R52: zusammen und unter Einschluß des Stickstoffatoms, an das beide gebunden sind, einen 5-, 6- oder 7-gliedrigen, gewünschtenfalls zusätzlich durch eine Gruppe -N(R7)- oder ein Sauerstoffatom unterbrochenen Kohlenwasserstoffring darstellen,
- R6: N(R61)R62 bedeutet, wobei R61 und R62 unabhängig voneinander Wasserstoff, 1-7C-Alkyl, 3-7C-Cycloalkyl oder 3-7C-Cycloalkylmethyl bedeuten,
- R7: Wasserstoff oder 1-4C-Alkyl bedeutet,
- m: für 2, 3 oder 4 steht,
- n: für 1, 2, 3 oder 4 steht,
- p: für 1, 2, 3 oder 4 steht,
sowie die Salze dieser Verbindungen.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-7C-Alkyl steht für geradkettige oder verzweigte Alkylreste mit 1 bis 7 Kohlenstoffatomen. Beispielsweise seien genannt der Heptyl-, Isoheptyl- (5-Methylhexyl-), Hexyl-, Isohexyl- (4-Methylpentyl-), Neohexyl- (3,3-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

1-40-Alkoxy steht für Reste, die neben dem Sauerstoffatom einen geradkettigen oder verzweigten Alkylrest mit 1 bis 4 Kohlenstoffatomen enthalten. Beispielsweise seien genannt der Butoxy-, iso-Butoxy-, sec.-Butoxy-, tert.-Butoxy-, Propoxy-, Isopropoxy- und bevorzugt der Ethoxy- und Methoxyrest.

3-7C-Cycloalkoxy steht für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder überwiegend durch Fluor substituiertes 1-4C-Alkoxy seien beispielsweise der 2,2,3,3,3-Pentafluorpropoxy-, der Perfluorethoxy-, der 1,2,2-Trifluorethoxy-, insbesondere der 1,1,2,2-Tetrafluorethoxy-, der 2,2,2-Trifluorethoxy-, der Trifluormethoxy- und bevorzugt der Difluormethoxyrest genannt.

Als spiro-verknüpfter 5-, 6- oder 7-gliedriger, gewünschtenfalls durch ein Sauerstoffatom unterbrochener Kohlenwasserstoffring sei beispielsweise der Cyclopentan-, der Cyclohexan-, der Cycloheptan-, der Tetrahydrofuran- und der Tetrahydropyranring genannt.

Als Reste -CₙH₂ₙ- und -CₚH₂ₚ- kommen geradkettige oder verzweigte Reste mit 1 bis 4 Kohlenstoffatomen in Frage. Beispielsweise seien genannt der Butylen-, iso-Butylen-, Propylen-, iso-Propylen-, Ethylen-, 1-Methylmethylen- und der Methylenrest.

Als Reste -CₘH₂ₘ- kommen geradkettige oder verzweigte Reste mit 2 bis 4 Kohlenstoffatomen in Frage. Beispielsweise seien genannt der Butylen-, iso-Butylen-, Propylen-, iso-Propylen-, Ethylen- und der 1 -Methylmethylenrest.

1-7C-Alkoxy steht für Reste, die neben dem Sauerstoffatom einen geradkettigen oder verzweigten Alkylrest mit 1 bis 7 Kohlenstoffatomen enthalten. Beispielsweise seien genannt der Heptyloxy-, Isoheptyloxy- (5-Methylhexyloxy-), Hexyloxy-, lsohexyloxy- (4-Methylpentyloxy-), Neohexyloxy- (3,3-Dimethylbutoxy-), Pentyloxy-, lsopentyloxy- (3-Methylbutoxy-), Neopentyloxy- (2,2-Dimethylpropoxy-), Butoxy-, iso-Butoxy-, sec.-Butoxy-, tert.-Butoxy-, Propoxy- und bevorzugt der Isopropoxy-, Ethoxy- und Methoxyrest.

Als Beispiele für die Gruppierung R51(R52)N-, in der R51 und R52 zusammen und unter Einschluß des Stickstoffatoms, an das beide gebunden sind, einen 5-, 6- oder 7-gliedrigen, gewünschtenfalls zusätzlich durch eine Gruppe -N(R7)- oder ein Sauerstoffatom unterbrochenen Kohlenwasserstoffring darstellen, seien der 4-Methyl-1-piperazinyl-, 1-Piperazinyl-, 1- Pyrrolidinyl-, 1-Piperidyl-, 1-Hexahydroazepinyl- und der 4-Morpholinylrest genannt.

3-7C-Cycloalkyl steht für Cyclopropyl, Cyclobutyl, Cyclopentyl, Cyclohexyl und Cycloheptyl, wovon Cyclopropyl, Cyclobutyl und Cyclopentyl bevorzugt sind.

3-7C-Cycloalkylmethyl steht für einen Methylrest, der durch einen der vorstehend genannten 3-7C-Cycloalkylreste substituiert ist. Bevorzugt seien die 3-5C-Cycloalkylmethylreste Cyclopropyimethyl, Cyclobutylmethyl und Cyclopentylmethyl genannt.

Als Salze kommen für Verbindungen der Formel l - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Megluminoder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel l, sowie alle Solvate und insbesondere alle Hydrate der Salze der Verbindungen der Formel I.

Hervorzuhebende Verbindungen der Formel l sind solche, worin
- Y: O (Sauerstoff) oder NH bedeutet,
- R1: 1-4C-Alkoxy, 3-5C-Cycloalkoxy, 3-5C-Cycloalkylmethoxy, oder ganz oder überwiegend durch Fluor substituiertes 1-2C-Alkoxy bedeutet,
- R2: 1-4C-Alkyl und
- R3: Wasserstoff oder 1-4C-Alkyl bedeutet,
oder worin
- R2 und R3: gemeinsam und unter Einschluß der beiden Kohlenstoffatome, an die sie gebunden sind, einen spiroverknüpften Cyclopentan-, Cyclohexan-, Tetrahydrofuran- oder Tetrahydropyranring darstellen,
- R4: C(O)R5, C(O)R6, -CₙH₂ₙ-C(O)R5 oder -CₙH₂ₙ-C(O)R6 bedeutet, wobei
- R5: Hydroxy, 1-7C-Alkoxy, 3-7C-Cycloalkoxy, 3-7C-Cycloalkylmethoxy, H₂N-Cₘ,H₂ₘ-O- oder R51(R52)N-CₚH₂ₚ-O- bedeutet, wobei
- R51: Wasserstoff oder 1-4C-Alkyl bedeutet und
- R52: 1-4C-Alkyl bedeutet, oder wobei
- R51 und R52: zusammen und unter Einschluß des Stickstoffatoms, an das beide gebunden sind, einen 5-, 6- oder 7-gliedrigen, gewünschtenfalls zusätzlich durch eine Gruppe -N(R7)- oder ein Sauerstoffatom unterbrochenen Kohlenwasserstoffring darstellen,
- R6: N(R61)R62 bedeutet, wobei R61 und R62 unabhängig voneinander Wasserstoff oder 1-4C-Alkyl bedeuten,
- R7: Wasserstoff oder 1-4C-Alkyl bedeutet,
- m: für 2, 3 oder 4 steht,
- n: für 1 oder 2 steht,
- p: für 1, 2, 3 oder 4 steht,
sowie die Salze dieser Verbindungen.

Besonders hervorzuhebende Verbindungen der Formel l sind solche, worin
- Y: O (Sauerstoff) oder NH bedeutet,
- R1: Methoxy, Ethoxy, Cyclopropylmethoxy oder ganz oder überwiegend durch Fluor substituiertes 1-2C-Alkoxy bedeutet,
- R2 und R3: gemeinsam und unter Einschluß der beiden Kohlenstoffatome, an die sie gebunden sind, einen spiroverknüpften Cyclopentan-, Cyclohexan-, Tetrahydrofuran- oder Tetrahydropyranring darstellen,
- R4: C(O)R5, C(O)R6, -CₙH₂ₙ-C(O)R5 oder -CₙH₂ₙ-C(O)R6 bedeutet, wobei
- R5: Hydroxy, 1 -4C-Alkoxy oder R51(R52)N-CₚH₂ₚ-O- bedeutet, wobei
- R51: Wasserstoff oder 1-4C-Alkyl bedeutet und
- R52: 1-4C-Alkyl bedeutet, oder wobei
- R51 und R52: zusammen und unter Einschluß des Stickstoffatoms, an das beide gebunden sind, einen 6-gliedrigen, gewünschtenfalls durch eine Gruppe -N(R7)- oder ein Sauerstoffatom unterbrochenen Kohlenwasserstoffring darstellen,
- R6: N(R61)R62 bedeutet, wobei R61 und R62 unabhängig voneinander Wasserstoff oder 1-4C-Alkyl bedeuten,
- R7: Wasserstoff oder 1-4C-Alkyl bedeutet,
- n: für 1 oder 2 steht,
- p: für 1, 2, 3 oder 4 steht,
sowie die Salze dieser Verbindungen.

Bevorzugte Verbindungen der Formel I sind solche, in denen
- Y: O (Sauerstoff) oder NH bedeutet,
- R1: Methoxy bedeutet,
- R2 und R3: gemeinsam und unter Einschluß der beiden Kohlenstoffatome, an die sie gebunden sind, einen spiroverknüpften Cyclopentan- oder Tetrahydropyranring darstellen,
- R4: Carboxyl, Carboxymethyl, Carboxymethylmethyl, Methoxycarbonylmethyl, Methoxycarbonylmethylmethyl, Methoxycarbonyl, Aminocarbonyl oder Morpholin-4-yl-ethylenoxy-carbonyl bedeutet,
sowie die Salze dieser Verbindungen.

Besonders bevorzugte Verbindungen der Formel l sind solche, in denen
- Y: O (Sauerstoff) oder NH bedeutet,
- R1: Methoxy bedeutet,
- R2 und R3: gemeinsam und unter Einschluß der beiden Kohlenstoffatome, an die sie gebunden sind, einen spiroverknüpften Cyclopentanring darstellen,
- R4: Carboxyl, Carboxymethyl, Carboxymethylmethyl, Methoxycarbonylmethyl, Methoxycarbonylmethylmethyl, Methoxycarbonyl, Aminocarbonyl oder Morpholin-4-yl-ethylenoxy-carbonyl bedeutet,
sowie die Salze dieser Verbindungen.

Beispielhafte erfindungsgemäße Verbindungen sind in den folgenden Tabellen aufgeführt:

**Tabelle 1**

| Verbindungen der Formel l mit Y=NH, R4=C(O)OH (in 5-Stellung des Benzimidazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 2**

| Verbindungen der Formel l mit Y=O R4=C(O)OH (in 5-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 3**

| Verbindungen der Formel l mit Y=O, R4=C(O) (in 6-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂O-CH₂ | |
| OCF₃ | CH₂CH₂-O-CH₂ | |

**Tabelle 4**

| Verbindungen der Formel l mit Y=NH, R4=C(O)OCH₃ (in 5-Stellung des Benzimidazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 5**

| Verbindungen der Formel l mit Y=O, R4=C(O)OCH₃ (in 5-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 6**

| Verbindungen der Formel l mit Y=O, R4=C(O)OCH₃ (in 6-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 7**

| Verbindungen der Formel l mit Y=NH, R4=C(O)NH₂ (in 5-Stellung des Benzimidazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH3 |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 8**

| Verbindungen der Formel l mit Y=O, R4=C(O)NH₂ (in 5-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

**Tabelle 9**

| Verbindungen der Formel l mit Y=O, R4=C(O)NH₂ (in 6-Stellung des Benzoxazol-Ringsystems) und den folgenden weiteren Substituentenbedeutungen: | | |
|---|---|---|
| **R1** | **R2** | **R3** |
| OCH₃ | CH₃ | H |
| OC₂H₅ | CH₃ | H |
| OCF₂H | CH₃ | H |
| OCF₃ | CH₃ | H |
| OCH₃ | C₂H₅ | CH₃ |
| OC₂H₅ | C₂H₅ | CH₃ |
| OCF₂H | C₂H₅ | CH₃ |
| OCF₃ | C₂H₅ | CH₃ |
| OCH₃ | CH₂CH₂CH₂ | |
| OC₂H₅ | CH₂CH₂CH₂ | |
| OCF₂H | CH₂CH₂CH₂ | |
| OCF₃ | CH₂CH₂CH₂ | |
| OCH₃ | CH₂CH₂CH₂CH₂ | |
| OC₂H₅ | CH₂CH₂CH₂CH₂ | |
| OCF₂H | CH₂CH₂CH₂CH₂ | |
| OCF₃ | CH₂CH₂CH₂CH₂ | |
| OCH₃ | CH₂-O-CH₂ | |
| OC₂H₅ | CH₂-O-CH₂ | |
| OCF₂H | CH₂-O-CH₂ | |
| OCF₃ | CH₂-O-CH₂ | |
| OCH₃ | CH₂CH₂-O | |
| OC₂H₅ | CH₂CH₂-O | |
| OCF₂H | CH₂CH₂-O | |
| OCF₃ | CH₂CH₂-O | |
| OCH₃ | CH₂CH₂-O-CH₂ | |
| OC₂H₅ | CH₂CH₂-O-CH₂ | |
| OCF₂H | CH₂CH₂-O-CH₂ | |
| OCF₃ | CH₂CH₂-O-CH₂ | |

sowie die Salze der in den Tabellen genannten Verbindungen.

Bei den Verbindungen der Formel l kann es sich, wenn Y NH bedeutet, um Tautomeren und - sofern die Substituenten -R2 und -CH₂R3 nicht identisch sind - um chirale Verbindungen handeln. Die Erfindung umfaßt daher sowohl die reinen Tautomeren und Enantiomeren als auch deren Gemische in jedem Mischungsverhältnis, einschließlich der Racemate. Die Enantiomeren können in an sich bekannter Weise (zum Beispiel durch Herstellung und Trennung entsprechender diastereoisomerer Verbindungen) separiert werden.

Bevorzugt sind jedoch die Verbindungen der Formel I, in denen die Substituenten -R2 und -CH₂R₃ identisch sind oder in denen R2 und R3 gemeinsam und unter Einschluß der beiden Kohlenstoffatome, an die sie gebunden sind, einen spiro-verknüpften 5-, 6- oder 7-gliedrigen Kohlenwasserstoffring darstellen. Bevorzugt sind außerdem die Verbindungen der Formel I, in denen R2 und R3 gemeinsam und unter Einschluß der beiden Kohlenstoffatome, an die sie gebunden sind, einen spiro-verknüpften 4'-Tetrahydropyranring darstellen.

Der Substituent R4 kann am Benzimidazol- oder -oxazol-Ringsystem in 4-, 5-, 6- oder 7-Stellung angebunden sein; bevorzugt sind jedoch die Verbindungen der Formel l, in denen der Substituent in 5-oder 6-Stellung angebunden ist.

Weiterer Gegenstand der Erfindung ist ein Verfahren zur Herstellung der Verbindungen der Formel l und ihrer Salze. Das Verfahren (vgl. Schema 1) ist dadurch gekennzeichnet, daß man Verbindungen der Formel II, in denen R1, R2 und R3 die oben angegebenen Bedeutungen haben und Z eine geeignete Abgangsgruppe darstellt, mit Verbindungen der Formel lll, worin Y O (Sauerstoff) oder NH bedeutet, und R4 die oben angegebenen Bedeutungen hat, umsetzt.

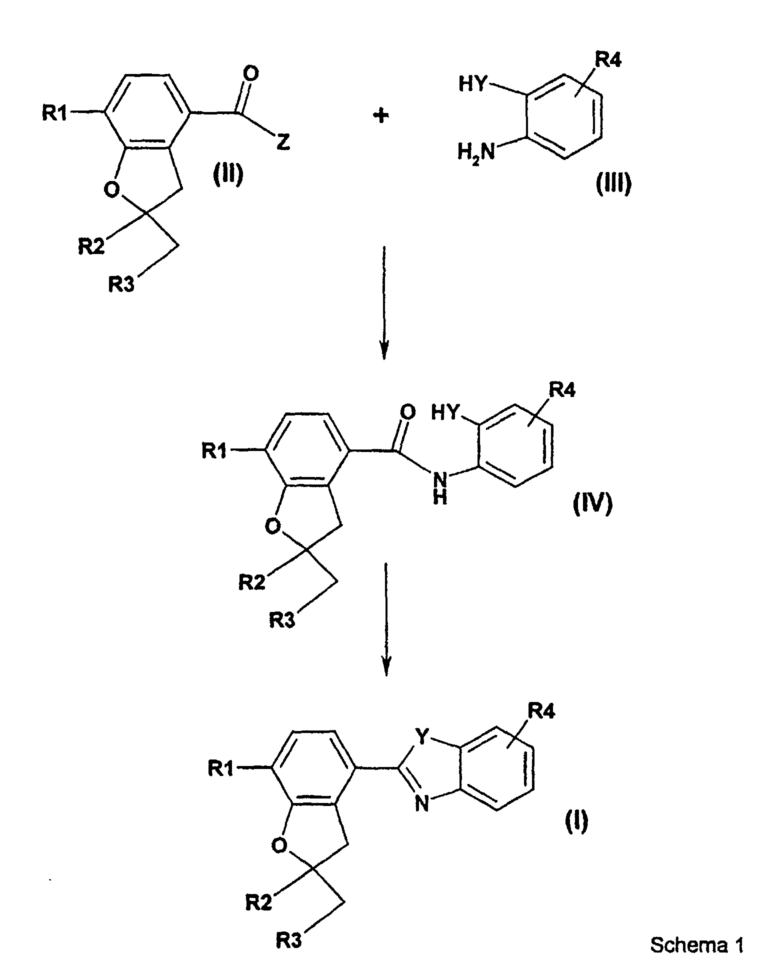

Welche Abgangsgruppen Z geeignet sind, ist dem Fachmann aufgrund seines Fachwissens geläufig. Beispielsweise wird von geeigneten Säurehalogeniden der Formel ll (Z = Cl oder Br) ausgegangen.

Die Umsetzung der Verbindungen der Formel ll mit Verbindungen der Formel lll erfolgt bevorzugt in Gegenwart einer Base wie z.B. Pyridin oder Triethylamin, in einem geeigneten inerten Lösungsmittel, z.B. in einem cyclischen Kohlenwasserstoff wie Toluol oder Xylol, oder einem sonstigen inerten Lösungsmittel wie Dioxan oder ohne weiteres Lösungsmittel, vorzugsweise bei erhöhter Temperatur.

Die bei der Umsetzung zunächst entstehenden Verbindungen der Formel IV, worin R1, R2, R3, R4 und Y die oben angegebenen Bedeutungen besitzen, werden durch innere Kondensation zu entsprechenden Verbindungen der Formel l umgesetzt. Diese innere Kondensation kann beispielsweise thermisch durch einfaches Erhitzen erfolgen; bevorzugt erfolgt sie jedoch in Gegenwart eines geeigneten Kondensationsmittels, wie beispielsweise Thionylchlorid oder Phosphoroxytrichlorid, in einem geeigneten inerten Lösungsmittel oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Die Umsetzung erfolgt beispielsweise so wie in den nachfolgenden Beispielen beschrieben, oder auf eine dem Fachmann an sich vertraute Weise, z.B. so wie in der internationalen Anmeldung WO94/12461 beschrieben.

Die erhaltenen Verbindungen der Formel l können anschließend in ihre Salze, gegebenenfalls erhaltene Salze der Verbindungen der Formel l in freie Verbindungen übergeführt werden.

Verbindungen der Formel ll, worin Z eine geeignete Abgangsgruppe darstellt und R1, R2 und R3 die oben angegebenen Bedeutungen besitzen, lassen sich wie in den folgenden Beispielen beschrieben oder in einer dem Fachmann an sich vertrauten Weise aus den entsprechenden Verbindungen der Formel ll, worin Z eine Hydroxygruppe bedeutet und R1, R2 und R3 die oben angegebenen Bedeutungen besitzen, herstellen.

Verbindungen der Formel II, worin Z eine Hydroxygruppe bedeutet und R1, R2 und R3 die oben angegebenen Bedeutungen besitzen, können wie in WO96/03399 beschrieben oder nach dem Fachmann vertrauten Methoden und Techniken erhalten werden.

Verbindungen der Formel lll sind bekannt oder können in einer dem Fachmann an sich vertrauten Weise unter Anwendung üblicher Verfahren hergestellt werden.

Dem Fachmann ist außerdem bekannt, daß es im Fall mehrerer reaktiver Zentren an einer Ausgangsoder Zwischenverbindung notwendig sein kann, ein oder mehrere reaktive Zentren temporär durch Schutzgruppen zu blockieren, um eine Reaktion gezielt am gewünschten Reaktionszentrum ablaufen zu lassen. Eine ausführliche Beschreibung zur Anwendung einer Vielzahl bewährter Schutzgruppen findet sich beispielsweise in T.W. Greene, Protective Groups in Organic Synthesis, John Wiley & Sons, 1991.

Gewünschtenfalls können erhaltene Verbindungen der Formel l durch Derivatisierung in weitere Verbindungen der Formel l übergeführt werden. Auf diese Weise können beispielsweise aus Verbindungen der Formel l, worin R1, R2 und R3 die oben angegebenen Bedeutungen besitzen und R4 eine Estergruppe beinhaltet, durch saure oder alkalische Verseifung die entsprechenden Säuren (R4 = -CₙH₂ₙ-COOH, COOH) erhalten werden oder durch Umsetzung mit Aminen der Formel HN(R61)R62, worin R61 und R62 die oben angegebenen Bedeutungen besitzen, die entsprechenden Amide dargestellt werden. Die Umsetzungen erfolgen zweckmäßigerweise analog dem Fachmann bekannter Methoden z.B. so wie in den nachfolgenden Beispielen beschrieben.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel (z. B. einem Keton, wie Aceton, Methylethylketon oder Methylisobutylketon, einem Ether, wie Diethylether, Tetrahydrofuran oder Dioxan, einem chlorierten Kohlenwasserstoff, wie Methylenchlorid oder Chloroform, oder einem niedermolekularen aliphatischen Alkohol wie Ethanol oder Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die nachfolgenden Beispiele dienen der näheren Erläuterung der Erfindung ohne sie einzuschränken. Ebenso können weitere Verbindungen der Formel l, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmeizpunkt, h für Stunde(n), min für Minute(n), RT für Raumtemperatur, SF für Summenformel und MG für Molgewicht. Die in den Beispielen genannten Verbindungen und ihre Salze sind bevorzugter Gegenstand der Erfindung.

### Beispiele

### Endprodukte

### 1. 2-(2,3-Dihydro-7-methoxy-benzofuran-2-spiro-1'-cyclopentan-4-yl)-benzimidazol-5-carbonsäure

3.44 g (9.1 mmol) 2-(2,3-Dihydro-7-methoxy-benzofuran-2-spiro-1'-cyclopentan-4-yl-benzimidazol-5-carbonsäuremethylester (Verbindung 13) werden in 50 ml 4 N Natronlauge 3 h zum Rückfluß erhitzt. Man läßt abkühlen, verdünnt mit 100 ml Wasser und tropft unter Rühren langsam 100 ml 2 N Salzsäure zu. Das Produkt wird abfiltriert und im Hochvakuum getrocknet. Man erhält 3.06 g der Titelverbindung vom Schmp. 235-240°C.

### 2. 2-(2,3-Dihydro-7-methoxy-benzofuran-2-spiro-1'-cyclopentan-4-yl)-benzimidazol-5-carbon säureamid

500 mg (1.37 mmol) 2-(2,3-Dihydro-7-methoxy-benzofuran-2-spiro-1'-cyclopentan-4-yl)-benzimidazol-5-carbonsäure (Verbindung 1) werden in 10 ml Thionylchlorid 45 min zum Rückfluß erhitzt. Man entfemt überschüssiges Thionylchlorid Im Vakuum und koevaporiert mehrmals mit Toluol. Der Rückstand wird in 5 ml Aceton suspendiert und unter Eiskühlung 5 ml konzentrierter Ammoniak zugetropft. Anschließend läßt man auf RT kommen. Nach Reaktionsende (DC-Kontrolle) verdünnt man mit 50 ml Wasser und extrahiert mit Ethylacetat. Die organischen Phasen werden gesammelt, über Magnesiumsulfat getrocknet und eingeengt. Der Rückstand wird aus Methanol kristallisiert und man erhält 180 mg der Titelverbindung vom Schmp. 245°C.

### 3. 2-(2,3-Dihydro-7-methoxy-benzofuran-2-spiro-1'-cyclopentan-4-yl)-benzoxazol-5-carbonsäure

Eine Suspension von 120 mg (0.32 mmol) 2-(2,3-Dihydro-7-methoxy-benzofuran-2-spiro-1'-cyclopentan-4-yl)-benzoxazol-5-carbonsäuremethylester (Verbindung 14) in einem Gemisch aus 10 ml Wasser und 10 ml Ethanol wird mit 40 mg (0.83 mmol) Lithiumhydroxid versetzt und 24 h bei 60°C gerührt. Man neutralisiert mit halbkonzentrierter Salzsäure und extrahiert mit 2 x 20 ml Ethylacetat. Die vereinigten organischen Phasen werden über Magnesiumsulfat getrocknet und bis zur beginnenden Kristallisation eingeengt. Der Kristallbrei wird abgesaugt und getrocknet und man erhält 70 mg der Titelverbindung vom Schmp. > 250°C.

### 4. 2-(2,3-Dihydro-7-methoxy-benzofuran-2-spiro-1'-cyclopentan-4-yl)-benzoxazol-5 yl-essigsäure

100 mg (0.25 mmol) 2-(2,3-Dihydro-7-methoxy-benzofuran-2-spiro-1'-cyclopentan-4-yl)-benzoxazol-5-yl-essigsäuremethylester (Verbindung 15) und 30 mg Lithiumhydroxid (1.25 mmol) werden in einem Gemisch aus 5 ml Wasser und 5 ml Ethanol über Nacht bei RT gerührt. Man verdünnt mit 10 ml Wasser und versetzt mit 1 ml 2 N Salzsäure. Nach Extraktion mit 2 x 30 ml Ethylacetat trocknet man die vereinigten organischen Phasen über Magnesiumsulfat, engt ein und rührt den Rückstand in Diethylether aus. Man erhält 80 mg der Titelverbindung vom Schmp. 220°C.

### 5. 2-[2-(2,3-Dihydro-7-methoxy-benzofuran-2-spiro-1'-cyclopentan-4-yl)-benzoxazol-5-yl]-propionsäure

Zu einer Lösung von 200 mg (0.53 mmol) 2-[2-(2,3-Dihydro-7-methoxy-benzofuran-2-spiro-1'-cyclopentan-4-yl)-benzoxazol-5-yl]-propionitril (Ausgangsverbindung A1) in 50 ml Methanol wird unter Eiskühlung bis zur Sättigung Chlorwasserstoff eingeleitet. Die Reaktionslösung wird 5 Tage im Kühlschrank stehengelassen. Dann wird die methanolische Salzsäurelösung im Vakuum entfernt und zweimal mit Toluol koevaporiert. Man nimmt in 10 ml Glycerin auf, versetzt mit 500 mg Kaliumhydroxid und erhitzt 1 h auf 160°C. Anschließend wird mit 20 ml 1 N Salzsäure angesäuert, mit Ethylacetat extrahiert, die organische Phase mit gesättigter Natriumchlorid-Lösung gewaschen, über Magnesiumsulfat getrocknet und eingeengt. Der Rückstand wird in 10 ml 0.1 N Natronlauge gelöst, die Lösung mit Kieselguhr geklärt und das Produkt mit 15 ml 0.1 N Salzsäure ausgefällt. Man erhält 145 mg der Titelverbindung vom Schmp. 90-105°C (Zers.).

### 6. 2-(2,3-Dihydro-7-methoxy-benzofuran-2-spiro-4'-tetrahydropyran-4-yl)-benzoxazol-5-carbonsäure

Eine Suspension von 1.16 g (2.8 mmol) 2-(2,3-Dihydro-7-methoxy-benzofuran-2-spiro-4'-tetrahydropyran-4-yl)-benzoxazol-5-carbonsäuremethylester (Verbindung 16) in einem Gemisch aus 100 ml Wasser und 20 ml Ethanol wird mit 540 mg (22.4 mmol) Lithiumhydroxid versetzt und 48 h bei RT gerührt. Das Ethanol wird eingeengt, weitere 30 ml Wasser zugesetzt und unter Rühren und Eiskühlung durch Zutropfen von 2 N Salzsäure das Reaktionsgemisch auf pH = 1-2 angesäuert. Der Niederschlag wird abgesaugt und über Kaliumhydroxid getrocknet. Man erhält 1.04 g der Titelverbindung vom Schmp. 251-253°C.

### 7. 2-(2,3-Dihydro-7-methoxy-benzofuran-2-spiro-4'-tetrahydropyran-4-yl)-benzoxazol-5-carbonsäureamid

300 mg (0.69 mmol) 2-(2,3-Dihydro-7-methoxy-benzofuran-2-spiro-4'-tetrahydropyran-4-yl)-benzoxazol-5-carbonsäure (Verbindung 6) werden in 1 ml Thionylchlorid 30 min zum Rückfluß erhitzt. Man entfernt überschüssiges Thionylchlorid im Vakuum und koevaporiert mehrmals mit Toluol. Der Rückstand wird in 20 ml Aceton suspendiert und unter Eiskühlung 6 ml konzentrierter Ammoniak zugetropft. Anschließend läßt man auf RT kommen. Nach Reaktionsende (DC-Kontrolle) wird das Aceton abdestilliert und das Reaktionsgemisch mit 10 ml Wasser verdünnt. Der Niederschlag wird abfiltriert, mit kaltem Wasser und kaltem Aceton gewaschen und über Kaliumhydroxid getrocknet. Man erhält 260 mg der Titelverbindung vom Schmp. 238-240°C.

### 8. 2-(2,3-Dihydro-7-methoxy-benzofuran-2-spiro-1'-cyclopentan-4-yl)-benzoxazol-6-carbonsäure

1.8 g (4.75 mmol) 2-(2,3-Dihydro-7-methoxy-benzofuran-2-spiro-1'-cyclopentan-4-yl)-benzoxazol-6-carbonsäuremethylester (Verbindung 17) werden in 30 ml 4N Natronlauge und 30 ml Methanol eine Stunde auf 80°C erhitzt. Man läßt abkühlen, verdünnt mit 300 ml Wasser und tropft unter Rühren langsam 20 ml halbkonzentrierte Salzsäure zu. Das Produkt wird abfiltriert und im Hochvakuum getrocknet. Man erhält 0.95 g der Titelverbindung vom Schmp. > 250°C.

### 9. 2-(2,3-Dihydro-7-methoxy-benzofuran-2-spiro-1'-cyclopentan-4-yl)-benzoxazol-6-carbonsäure-2-morpholin-4-yl-ethylester

1.5 g (4.1 mmol) 2-(2,3-Dihydro-7-methoxy-benzofuran-2-splro-1'-cyclopentan-4-yl)-benzoxazol-6-carbonsäure (Verbindung 8) werden in 10 ml Thionylchlorid 45 min zum Rückfluß erhitzt. Man läßt abkühlen, engt ein und koevaporiert mehrmals mit Toluol. Der Rückstand wird innerhalb 15 min portionsweise zu einer Lösung von 1.49 ml (12.3 mmol) N-(2-Hydroxyethyl)-morpholin und 33 µl (0.41 mmol) Pyridin in 30 ml Dioxan gegeben. Nach 20 min wird filtriert und das Filtrat eingeengt. Man chromatographiert den Rückstand über Kieselgel [Ethylacetat]. Das Produkt kristallisiert aus Acetonitril. Man erhält 1.49 g der Titelverbindung vom Schmp. 143-144°C.

### 10. 2-[2-(2,3-Dihydro-7-methoxy-benzofuran-2-spiro-1'-cyclopentan-4-yl)-benzoxazol-6-yl)-propionsäure

1.3 g (3 mmol) 2-{4-[(2,3-Dihydro-7-methoxy-benzofuran-2-spiro-1'-cyclopentan-4-carbonyl)-amino]-3-hydroxyphenyl}-propionsäureethylester (Ausgangsverbindung A8) werden 4 h auf 240°C erhitzt. Man chromatographiert das Reaktionsgemisch über Kieselgel [Toluol/Ethylacet = 5:1]. Das Produkt, 2-[2-(2,3-Dihydro-7-methoxy-benzofuran-2-spiro-1'-cyclopentan-4-yl)-benzoxazol-6-yl]-propionsäureethylester, wird in 20 ml Ethanol und 10 ml Wasser aufgenommen und die Lösung mit 240 mg (10 mmol) Lithiumhydroxid versetzt. Man rührt 16 h bei RT, destilliert das Ethanol weitgehend ab, und extrahiert mit Ethylacetat. Die vereinigten organischen Phasen werden über Magnesiumsulfat getrocknet, eingeengt und der Rückstand aus 5 ml Ethanol umkristallisiert. Man erhält 430 mg der Titelverbindung vom Schmp. 172-174°C.

### 11. 2-(2,3-Dihydro-7-methoxy-benzofuran-2-spiro-4'-tetrahydropyran-4-yl)-benzoxazol-6-car bonsäure

Eine Suspension von 1.36 g (3.29 mmol) 2-(2,3-Dihydro-7-methoxy-benzofuran-2-spiro-4'-tetrahydropyran-4-yl)-benzoxazol-6-carbonsäuremethylester (Verbindung 18) in einem Gemisch aus 100 ml Wasser und 20 ml Ethanol wird mit 630 mg (26.2 mmol) Lithiumhydroxid versetzt und 48 h bei RT gerührt. Das Ethanol wird eingeengt, weitere 30 ml Wasser zugesetzt und unter Rühren und Eiskühlung durch Zutropfen von 2 N Salzsäure das Reaktionsgemisch auf pH = 1-2 angesäuert. Der Niederschlag wird abgesaugt und über Kaliumhydroxid getrocknet. Man erhält 1.21 g der Titelverbindung vom Schmp. > 260°C.

### 12. 2-(2,3-Dihydro-7-methoxy-benzofuran-2-spiro-4'-tetrahydropyran-4-yl)-benzoxazol-6-car bonsäureamid

300 mg (0.79 mmol) 2-(2,3-Dihydro-7-methoxy-benzofuran-2-spiro-4'-tetrahydropyran-4-yl)-benzoxazol-6-carbonsäure (Verbindung 11) werden in 1 ml Thionylchlorid 30 min zum Rückfluß erhitzt. Man entfernt überschüssiges Thionylchlorid im Vakuum und koevaporiert mehrmals mit Toluol. Der Rückstand wird in 20 ml Aceton suspendiert und unter Eiskühlung 6 ml konzentrierter Ammoniak zugetropft. Der Niederschlag wird abfiltriert, mit kaltem Aceton gewaschen und über Kaliumhydroxid getrocknet. Man erhält 220 mg der Titelverbindung vom Schmp. 262-264°C.

### 13. 2-(2,3-Dihydro-7-methoxy-benzofuran-2-spiro-1'-cyclopentan-4-yl)-benzimidazol-5-carbonsäuremethylester

5.92 g (14.95 mmol) 3-Amino-4-[(2,3-dihydro-7-methoxy-benzofuran-2-spiro-1'-cyclopentan-4-carbonyl)-amino]-benzoesäuremethylester (Ausgangsverbindung A2) werden 90 min in 20 ml Thionylchlorid zum Rückfluß erhitzt. Das Reaktionsgemisch wird eingeengt und mehrmals mit Toluol koevaporiert. Man setzt 50 ml einer gesättigten Natriumhydrogencarbonat-Lösung zu, extrahiert mit 2 x 30 ml Dichlormethan, trocknet die vereinigten organischen Phasen über Magnesiumsulfat und engt ein. Das Produkt kristallisiert aus Ethylacetat. Man erhält 3.54 g der Titelverbindung vom Schmp. 203°C.

### 14. 2-(2,3-Dihydro-7-methoxy-benzofuran-2-spiro-1'-cyclopentan-4-yl)-benzoxazol-5-carbonsäuremethylester

2.96 g (7.45 mmol) 3-[(2,3-Dihydro-7-methoxy-benzofuran-4-spiro-1'-cyclopentan-4-carbonyl)-amino]-4-hydroxy-benzoesäuremethylester (Ausgangsverbindung A3) werden 3 h in 15 ml Thionylchlorid zum Rückfluß erhitzt. Das Reaktionsgemisch wird eingeengt und mehrmals mit Toluol koevaporiert. Man setzt 50 ml einer gesättigten Natriumhydrogencarbonat-Lösung zu, extrahiert mit 2 x 30 ml Dichlormethan, trocknet die vereinigten organischen Phasen über Magnesiumsulfat und engt ein. Das Produkt kristallisiert aus Ethylacetat. Man erhält 1.95 g der Titelverbindung vom Schmp. 167-169°C.

### 15. 2-(2,3-Dihydro-7-methoxy-benzofuran-2-spiro-1'-cyclopentan-4-yl)-benzoxazol-5-yl-essigsäuremethylester

1.1 g (2.8 mmol) 3-[(2,3-Dihydro-7-methoxy-benzofuran-2-spiro-1 '-cyclopentan-4-carbonyl)-amino]-4-hydroxy-phenylessigsäuremethylester (Ausgangsverbindung A4; Rohprodukt) werden 6 h in 10 ml Thionylchlorid zum Rückfluß erhitzt. Das Reaktionsgemisch wird eingeengt und mehrmals mit Toluol koevaporiert. Man setzt 25 ml 2 N Natronlauge zu, extrahiert mit 2 x 30 ml Dichlormethan, trocknet die vereinigten organischen Phasen über Magnesiumsulfat und engt ein. Das Produkt wird über Kieselgel [Toluol/Ethylacetat = 20:1] chromatographiert und aus Isopropanol kristallisiert. Man erhält 200 mg der Titelverbindung vom Schmp. 130-131°C.

### 16. 2-(2,3-Dihydro-7-methoxy-benzofuran-2-spiro-4'-tetrahydropyran-4-yl)-benzoxazol-5-carbonsäuremethylester

2.0 g (4.83 mmol) 3-[(2,3-Dihydro-7-methoxy-benzofuran-2-spiro-4'-tetrahydropyran-4-carbonyl)-amino]-4-hydroxy-benzoesäuremethylester (Ausgangsverbindung A6) werden 3 h in 15 ml Thionylchlorid auf 75°C erhitzt. Das Reaktionsgemisch wird eingeengt und mehrmals mit Toluol koevaporiert. Das Rohprodukt wird aus Acetonitril umkristallisiert. Man erhält 1.16 g der Titelverbindung vom Schmp. 180-181°C.

### 17. 2-(2,3-Dihydro-7-methoxy-benzofuran-2-spiro-1'-cyclopentan-4-yl)-benzoxazol-6-carbonsäuremethylester

750 mg (1.9 mmol) 4-[(2,3-Dihydro-7-methoxy-benzofuran-2-spiro-1'-cyclopentan-4-carbonyl)-amino]-3-hydroxy-benzoesäuremethylester (Ausgangsverbindung A7) werden 6 h in 4 ml Thionylchlorid zum Rückfluß erhitzt. Das Reaktionsgemisch wird eingeengt und mehrmals mit Toluol koevaporiert. Man setzt 10 ml 2 N NaOH zu, extrahiert mit 2 x 30 ml Dichlormethan, wäscht die vereinigten organischen Phasen mit 10 ml Wasser, dann mit 10 ml einer gesättigten Natriumchlorid-Lösung, trocknet über Magnesiumsulfat und engt ein. Das Produkt kristallisiert aus Ethylacetat. Man erhält 170 mg der Titelverbindung vom Schmp. 178°C.

### 18. 2-(2,3-Dihydro-7-methoxy-benzofuran-2-spiro-4'-tetrahydropyran-4-yl)-benzoxazol-6-car bonsäuremethylester

2.0 g (4.83 mmol) 4-[(2,3-Dihydro-7-methoxy-benzofuran-2-spiro-4'-tetrahydropyran-4-carbonyl)-amino]-3-hydroxy-benzoesäuremethylester (Ausgangsverbindung A9) werden 4.5 h in 15 ml Thionylchlorid auf 75°C erhitzt. Das Reaktionsgemisch wird eingeengt und mehrmals mit Toluol koevaporiert. Das Rohprodukt wird in Acetonitril umkristallisiert. Man erhält 1.36 g der Titelverbindung vom Schmp. 215-225°C.

### Ausgangsverbindungen:

### A1. 2-[2-(2,3-Dihydro-7-methoxy-benzofuran-2-spiro-1'-cyclopentan-4-yl)-benzoxazol-5- yl]-propionitril

800 mg (2.0 mmol) 2-{3-((2,3-Dihydro-7-methoxy-benzofuran-2-spiro-1' -cyclopentan-4-carbonyl)-amino]-4-hydroxyphenyl}-propionitril (Ausgangsverbindung A5) werden 1 h in 10 ml Thionylchlorid zum Rückfluß erhitzt. Das Reaktionsgemisch wird eingeengt und mehrmals mit Toluol koevaporiert. Der Rückstand wird über Kieselgel chromatographiert [Toluol] und das Produkt aus 5 ml Methanol kristallisiert. Män erhält 330 mg der Titelverbindung vom Schmp. 129-132°C.

### A2. 3-Amino-4-[(2,3-dihydro-7-methoxy-benzofuran-2-spiro-1'-cyclopentan-4-carbonyl)-ami-no]-benzoesäuremethylester

4.6 g (18.5 mmol) 2,3-Dihydro-7-methoxy-benzofuran-2-spiro-1'-cyclopentan-4-carbonsäure (Ausgangsverbindung A10) werden 45 min in 10 ml Thionylchlorid zum Rückfluß erhitzt. Anschließend engt man ein und koevaporiert mehrmals mit Toluol. Das Säurechlorid wird in 50 ml Dioxan gelöst und bei 40°C zu einer Lösung von 4.3 g (25.9 mmol) 3,4-Diaminobenzoesäuremethylester und 3.6 ml (25.9 mmol) Triethylamin in 100 ml Pyridin getropft. Man erhitzt das Reaktionsgemisch auf 60°C. Nach Reaktionsende (DC-Kontrolle) wird eingeengt und mehrmals mit Toluol koevaporiert. Der Rückstand wird zwischen Wasser und Ethylacetat verteilt. Das Produkt kristallisiert aus Toluol. Man erhält 4.74 g der Titelverbindung.

### A3. 3-[(2,3-Dihydro-7-methoxy-benzofuran-2-spiro-1'-cyclopentan-4-carbonyl-amino]-4-hydroxy-benzoesäuremethylester

1.5 g (6.1 mmol) 2,3-Dihydro-7-methoxy-benzofuran-2-spiro-1'-cyclopentan-4-carbonsäure (Ausgangsverbindung A10) werden 45 min in 5 ml Thionylchlorid zum Rückfluß erhitzt. Anschließend engt man ein und koevaporiert mehrmals mit Toluol. Das Säurechlorid wird in 40 ml Pyridin gelöst und die Lösung mit 1.16 g (6.96 mmol) 3-Amino-4-hydroxybenzoesäuremethylester versetzt. Man rührt über Nacht bei RT und erhitzt weitere 5 h auf 70°C. Nach Reaktionsende wird eingeengt und mehrmals mit Toluol koevaporiert. Der Rückstand wird über Kieselgel chromatographiert [Toluol/Ethylacetat = 9:1]. Das Produkt kristallisiert aus Methanol. Man erhält 820 mg der Titelverbindung.

### A4. 3-[(2,3-Dihydro-7-methoxy-benzofuran-2-spiro-1'-cyclopentan-4-carbonyl)-amino]-4-hydroxy-phenylessigsäuremethylester

690 mg (2.8 mmol) 2,3-Dihydro-7-methoxy-benzofuran-2-spiro-1'-cyclopentan-4-carbonsäure (Ausgangsverbindung A10) werden 60 min in 5 ml Thionylchlorid zum Rückfluß erhitzt. Anschließend engt man ein und koevaporiert mehrmals mit Toluol. Das Säurechlorid wird in 20 ml Pyridin gelöst und die Lösung mit 540 mg (2.64 mmol) 3-Amino-4-hydroxyphenylessigsäuremethylester (Ausgangsverbindung A12) versetzt. Man rührt über Nacht bei RT. Nach Reaktionsende wird eingeengt und mehrmals mit Toluol koevaporiert. Der Rückstand wird zwischen Wasser und Ethylacetat verteilt und die organische Phase über Magnesiumsulfat getrocknet. Das Rohprodukt wird ohne weitere Reinigung umgesetzt.

### A5. 2{3-[(2,3-Dihydro-7-methoxy-benzofuran-2-spiro-1'-cyclopentan-4-carbonyl)-amino]-4-hydroxyphenyl}-propionitril

985 mg (4.0 mmol) 2,3-Dihydro-7-methoxy-benzofuran-2-spiro-1'-cyclopentan-4-carbonsäure (Ausgangsverbindung A10) werden 2 h in 5 ml Thionylchlorid zum Rückfluß erhitzt. Anschließend engt man ein und koevaporiert mehrmals mit Toluol. Das Säurechlorid wird in 5 ml Dioxan gelöst und bei RT zu einer Lösung von 650 mg (4.0 mmol) 2-(3-Amino-4-hydroxyphenyl)-propionitril (Ausgangsverbindung A13) und 0.34 ml (4.2 mmol) Pyridin in 5 ml Dioxan getropft. Nach 3 h Rühren bei RT wird eingeengt und das Produkt aus 5 ml Methanol kristallisiert. Man erhält 1.18 g der Titelverbindung vom Schmp. 151-153°C.

### A6. 3-[(2,3-Dihydro-7-methoxy-benzofuran-2-spiro-4'-tetrahydropyran-4-carbonyl)-amino]-4-hydroxybenzoesäuremethylester

2.22 g (8.4 mmol) 2,3-Dihydro-7-methoxy-benzofuran-2-spiro-4'-tetrahydropyran-4-carbonsäure (Ausgangsverbindung A11) werden 75 min in 10 ml Thionylchlorid zum Rückfluß erhitzt. Anschließend engt man ein und koevaporiert mehrmals mit Toluol. Das Säurechlorid wird in 40 ml Dioxan gelöst und bei 10°C zu einer Lösung von 1.4 g (8.4 mmol) 3-Amino-4-hydroxybenzoesäuremethylester und 0.68 ml (8.4 mmol) Pyridin in 20 ml Dioxan getropft. Nach 90 min Rühren bei RT wird eingeengt und das Produkt aus 100 ml Methanol kristallisiert. Man erhält 2.6 g der Titelverbindung vom Schmp. 258-260°C.

### A7. 4-[(2,3-Dihydro-7-methoxy-benzofuran-2-spiro-1'-cyclopentan-4-carbonyl)-aminol-3-hydroxybenzoesäuremethylester

4.0 g (16.1 mmol) 2,3-Dihydro-7-methoxy-benzofuran-2-spiro-1'-cyclopentan-4-carbonsäure (Ausgangsverbindung A10) werden 60 min in 10 ml Thionylchlorid zum Rückfluß erhitzt. Anschließend engt man ein und koevaporiert mehrmals mit Toluol. Das Säurechlorid wird in 80 ml Pyridin gelöst und die Lösung mit 3.1 g (18.6 mmol) 4-Amino-3-hydroxybenzoesäuremethylester versetzt. Man rührt 4 h bei RT und erwärmt weitere 5 h auf 70°C. Nach Reaktionsende wird eingeengt und mehrmals mit Toluol koevaporiert. Der Rückstand wird zwischen Wasser und Ethylacetat verteilt und die organische Phase über Magnesiumsulfat getrocknet Das Produkt kristallisiert beim Abdampfen des Lösungsmittels und man erhält 2.96 g der Titelverbindung vom Schmp. > 230°C.

### A8. 2-{4-[(2,3-Dihydro-7-methoxy-benzofuran-2-spiro-1'-cyclopentan-4-carbonyl)-amino]-3-hydroxyphenyl}-propionsäureethylester

2.48 g (10.0 mmol) 2,3-Dihydro-7-methoxy-benzofuran-2-spiro-1'-cyclopentan-4-carbonsäure (Ausgangsverbindung A10) werden 1 h in 5 ml Thionylchlorid zum Rückfluß erhitzt. Anschließend engt man ein und koevaporiert mehrmals mit Toluol. Das Säurechlorid wird in 20 ml Dioxan gelöst und bei < 20°C zu einer Lösung von 2.1 g (10.0 mmol) 2-(4-Amino-3-hydroxyphenyl)-propionsäureethylester (Ausgangsverbindung A14) und 1.2 ml (15.0 mmol) Pyridin in 20 ml Dioxan getropft. Man rührt 2 Tage bei RT. Nach Reaktionsende wird eingeengt und mehrmals mit Toluol koevaporiert. Der Rückstand wird über Kieselgel chromatographiert [Toluol/Ethylacetat = 4:1]. Das Produkt wird aus 20 ml Methanol kristallisiert. Man erhält 3.1 g der Titelverbindung vom Schmp. 170-172°C.

### A9. 4-[(2,3-Dihydro-7-methoxy-benzofuran-2-spiro-4'-tetrahydropyran-4-carbonyl)-amino]-3-hydroxybenzoesäuremethylester

2.34 g (9.0 mmol) 2,3-Dihydro-7-methoxy-benzofuran-2-spiro-4'-tetrahydropyran-4-carbonsäure (Ausgangsverbindung A11) werden 75 min in 10 ml Thionylchlorid zum Rückfluß erhitzt. Anschließend engt man ein und koevaporiert mehrmals mit Toluol. Das Säurechlorid wird in 40 ml Dioxan gelöst und bei 10°C zu einer Lösung von 1.51 g (9.0 mmol) 4-Amino-3-hydroxybenzoesäuremethylester und 0.73 ml (9.0 mmol) Pyridin in 80 ml Dioxan getropft. Nach 90 min Rühren bei RT wird eingeengt und das Produkt in 100 ml heißem Methanol ausgerührt. Man erhält 3.27 g der Titelverbindung vom Schmp. > 260°C.

### A10. 2,3-Dihydro-7-methoxy-benzofuran-2-spiro-1'-cyclopentan-4-carbonsäure

Die Herstellung der Titelverbindung ist in WO96/03399 beschrieben.

### A11. 2,3-Dihydro-7-methoxy-benzofuran-2-spiro-4'-tetrahydropyran-4-carbonsäure

Die Herstellung der Titelverbindung ist in WO96/0339 beschrieben.

### A12. 3-Amino-4-hydroxy-phenylessigsäuremethylester

Lit.: D.R. Shridhar et.al.; Indian J. Chem. Sect. B; 20 (1981) 311-313

### A13. 2-(3-Amino-4-hydroxyphenyl)-propionitril

Lit.: D.W. Dunwell, D. Evans, T.A. Hicks; J. Med. Chem. 18 (1975) 53-58

### A14. 2-(4-Amino-3-hydroxyphenyl)-propionsäureethylester

Lit.: D.W. Dunwell, D. Evans; J. Med. Chem. 20 (1977) 797-801

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs 4) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des zentralen Nervensystems, des Darms, der Augen und der Gelenke, die vermittelt werden durch Mediatoren, wie Histamin, PAF (Plättchen-aktivierender Faktor), Arachidonsäure-Abkömmlinge wie Leukotriene und Prostaglandine, Zytokine, Interleukine, Chemokine, alpha-, beta- und gamma-lnterferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale, Emphysema, COPD); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Graft versus Host Reaktionen, Transplantationsabstoßungsreaktionen, Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen. Desweiteren können die erfindungsgemäßen Verbindungen zur Behandlung von Diabetes Insipidus und Erkrankungen im Zusammenhang mit Störungen des Hirnstoffwechsels, wie z. B. zerebrale Senilität, Senile Demenz (Alzheimer Demenz), Multiinfarkt Demenz oder auch Erkrankungen des ZNS, wie beispielsweise Depressionen oder arteriosklerotische Demenz eingesetzt werden.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe von Krankheiten, insbesondere den genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Ein weiterer Gegenstand der Erfindung ist ein Handelsprodukt, bestehend aus einem üblichen Sekundärpackmittel, einem das Arzneimittel enthaltenden Primärpackmittel (beispielsweise eine Ampulle oder ein Blister) und gewünschtenfalls einen Beipackzettel, wobei das Arzneimittel antagonistische Wirkung gegen zyklisch-nukleotid Phosphodiesterasen des Typs 4 (PDE4) zeigt und zur Abschwächung der Symptome von Krankheiten führt, die in Zusammenhang mit zyklisch-nukleotid Phosphodiesterasen des Typs 4 stehen, und wobei auf dem Sekundärpackmittel oder auf dem Beipackzettel des Handelsprodukts auf die Eignung des Arzneimittels zur Prophylaxe oder Behandlung von Krankheiten, die im Zusammenhang mit zyklisch-nukleotid Phosphodiesterasen des Typs 4 stehen, hingewiesen wird, und wobei das Arzneimittel ein oder mehrere erfindungsgemäße Verbindungen der Formel l enthält. Das Sekundärpackmittel, das das Arzneimittel enthaltende Primärpackmittel und der Beipackzettel entsprechen ansonsten dem, was der Fachmann als Standard für Arzneimittel dieser Art ansehen würde.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,1 und 3 mg pro Tag. Die übliche Dosis bei systemischer Therapie (p. o. oder i. v.) liegt zwischen 0,03 und 3 mg pro Kilogramm und Tag.

### Biologische Untersuchungen

Bei der Untersuchung der PDE 4-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminolverstärkte Chemilumineszenz gemessen werden kann. [Mc Phail LC, Strum SL, Leone PA und Sozzani S, The neutrophil respiratory burst mechanism. In "Immunology Series" **1992**, 57, 47-76; ed. Coffey RG (Marcel Decker, Inc., New York-Basel-Hong Kong)].

Substanzen, welche die Chemilumineszenz sowie die Zytokinsekretion und die Sekretion entzündungssteigernder Mediatoren an Entzündungszellen, insbesonders neutrophilen und eosinophilen Granulozyten, T-Lymphozyten, Monozyten und Makrophagen hemmen, sind solche, welche die PDE 4 hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE 4-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase Inhibitors render bronchodilatory therapy redundant in the treatment of bronchial asthma?. Biochem Pharmacol **1992**, 43, 2041-2051; **Torphy TJ** et al., Phosphodiesterase inhibitors: new opportunities for treatment of asthma. Thorax **1991**, 46, 512-523; **Schudt C** et al., Zardaverine: a cyclic AMP PDE 3/4 Inhibitor. In "New Drugs for Asthma Therapy", 379-402, Birkhäuser Verlag Basel 1991; Schudt C et al., Influence of selective phosphodiesterase inhibitors on human neutrophil functions and levels of cAMP and Ca; Naunyn-Schmiedebergs Arch Pharmacol 1991, 344, 682-690; **Tenor H** und **Schudt C**, Analysis of PDE isoenzyme profiles in cells and tissues by pharmacological methods. In "Phosphodiesterase Inhibitors", 21-40, "The Handbook of Immunopharmacology", Academic Press, 1996; **Hatzelmann A** et al., Enzymatic and functional aspects of dualselective PDE3/4-inhibitors. In "Phosphodiesterase Inhibitors", 147-160, "The Handbook of Immunopharmacology". Academic Press, 1996.

### Hemmung der PDE4-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980**, 311, 193-198). Hierbei erfolgt im ersten Schritt die PDE-Reaktion. In einem zweiten Schritt wird das entstandene 5'-Nukleotid durch eine 5'-Nukleotidase des Schlangengiftes von Crotalus Atrox zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorflüssigkelt zur Zählung gegeben wird.

Die für die erfindungsgemäßen Verbindungen ermittelten Hemmwerte [Hemmkonzentration als -log IC₅₀ (mol/l)] ergeben sich aus der folgenden Tabelle A, in der die Nummern der Verbindungen den Nummern der Beispiele entsprechen.

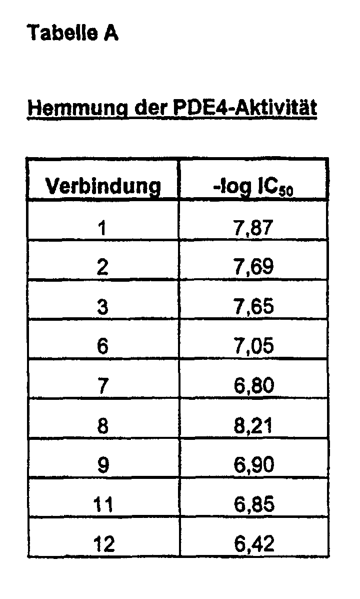

## Patentansprüche

1. Verbindungen der Formel l, 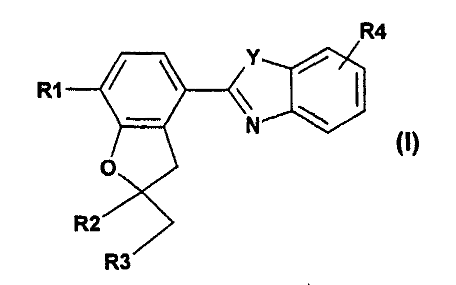 worin
Y O (Sauerstoff) oder NH bedeutet,
R1 1-4C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
R2 Wasserstoff oder 1-4C-Alkyl und
R3 Wasserstoff oder 1-4C-Alkyl bedeuten,
oder worin
R2 und R3 gemeinsam und unter Einschluß der beiden Kohlenstoffatome, an die sie gebunden sind, einen spiro-verknüpften 5-, 6- oder 7-gliedrigen, gewünschtenfalls durch ein Sauerstoffatom unterbrochenen Kohlenwasserstoffring darstellen,
R4 C(O)R5, C(O)R6, -CₙH₂ₙ-C(O)R5 oder -CₙH₂ₙ-C(O)R6 bedeutet, wobei
R5 Hydroxy, 1-7C-Alkoxy, 3-7C-Cycloalkoxy, 3-7C-Cycloalkylmethoxy, H₂N-CₘH₂ₘ-O- oder R51(R52)N-CₚH₂ₚ-O- bedeutet, wobei
R51 Wasserstoff, 1-7C-Alkyl, 3-7C-Cycloalkyl oder 3-7C-Cycloalkylmethyl bedeutet und
R52 1-7C-Alkyl, 3-7C-Cycloalkyl oder 3-7C-Cycloalkylmethyl bedeutet, oder wobei
R51 und R52 zusammen und unter Einschluß des Stickstoffatoms, an das beide gebunden sind, einen 5-, 6- oder 7-gliedrigen, gewünschtenfalls zusätzlich durch eine Gruppe -N(R7)- oder ein Sauerstoffatom unterbrochenen Kohlenwasserstoffring darstellen,
R6 N(R61)R62 bedeutet, wobei R61 und R62 unabhängig voneinander Wasserstoff, 1-7C-Alkyl, 3-7C-Cycloalkyl oder 3-7C-Cycloalkylmethyl bedeuten,
R7 Wasserstoff oder 1-4C-Alkyl bedeutet,
m für 2, 3 oder 4 steht,
n für 1, 2, 3 oder 4 steht,
p für 1, 2, 3 oder 4 steht,
sowie die Salze dieser Verbindungen.

2. Verbindungen der Formel l nach Anspruch 1, in denen
Y O (Sauerstoff) oder NH bedeutet,
R1 1-4C-Alkoxy, 3-5C-Cycloalkoxy, 3-5C-Cycloalkylmethoxy, oder ganz oder überwiegend durch Fluor substituiertes 1-2C-Alkoxy bedeutet,
R2 1-4C-Alkyl und
R3 Wasserstoff oder 1-4C-Alkyl bedeutet,
oder worin
R2 und R3 gemeinsam und unter Einschluß der beiden Kohlenstoffatome, an die sie gebunden sind, einen spiroverknüpften Cyclopentan-, Cyclohexan-, Tetrahydrofuran- oder Tetrahydropyranring darstellen,
R4 C(O)R5, C(O)R6, -CₙH₂ₙ-C(O)R5 oder -CₙH₂ₙ-C(O)R6 bedeutet, wobei
R5 Hydroxy, 1-7C-Alkoxy, 3-7C-Cycloalkoxy, 3-7C-Cycloalkylmethoxy, H₂N-CₘH₂ₘ-O- oder R51(R52)N-CₚH₂ₚ-O- bedeutet, wobei
R51 Wasserstoff oder 1-4C-Alkyl bedeutet und
R52 1-4C-Alkyl bedeutet, oder wobei
R51 und R52 zusammen und unter Einschluß des Stickstoffatoms, an das beide gebunden sind, einen 5-, 6- oder 7-gliedrigen, gewünschtenfalls zusätzlich durch eine Gruppe -N(R7)- oder ein Sauerstoffatom unterbrochenen Kohlenwasserstoffring darstellen,
R6 N(R61)R62 bedeutet, wobei R61 und R62 unabhängig voneinander Wasserstoff oder 1-4C-Alkyl bedeuten,
R7 Wasserstoff oder 1-4C-Alkyl bedeutet,
m für 2, 3 oder 4 steht,
n für 1 oder 2 steht,
p für 1, 2, 3 oder 4 steht,
sowie die Salze dieser Verbindungen.

3. Verbindungen der Formel I nach Anspruch 1, in denen
Y O (Sauerstoff) oder NH bedeutet,
R1 Methoxy, Ethoxy, Cyclopropylmethoxy oder ganz oder überwiegend durch Fluor substituiertes 1-2C-Alkoxy bedeutet,
R2 und R3 gemeinsam und unter Einschluß der beiden Kohlenstoffatome, an die sie gebunden sind, einen spiroverknüpften Cyclopentan-, Cyclohexan-, Tetrahydrofuran- oder Tetrahydropyranring darstellen,
R4 C(O)R5, C(O)R6, -CₙH₂n-C(O)R5 oder -CₙH₂ₙ-C(O)R6 bedeutet, wobei
R5 Hydroxy, 1 -4C-Alkoxy oder R51(R52)N-CₚH₂ₚ-O- bedeutet, wobei
R51 Wasserstoff oder 1-4C-Alkyl bedeutet und
R52 1-4C-Alkyl bedeutet, oder wobei
R51 und R52 zusammen und unter Einschluß des Stickstoffatoms, an das beide gebunden sind, einen 6-gliedrigen, gewünschtenfalls durch eine Gruppe -N(R7)- oder ein Sauerstoffatom unterbrochenen Kohlenwasserstoffring darstellen,
R6 N(R61)R62 bedeutet, wobei R61 und R62 unabhängig voneinander Wasserstoff oder 1-4C-Alkyl bedeuten,
R7 Wasserstoff oder 1-4C-Alkyl bedeutet,
n für 1 oder 2 steht,
p für 1, 2, 3 oder 4 steht,
sowie die Salze dieser Verbindungen.

4. Verbindungen der Formel l nach Anspruch 1, in denen
Y O (Sauerstoff) oder NH bedeutet,
R1 Methoxy bedeutet,
R2 und R3 gemeinsam und unter Einschluß der beiden Kohlenstoffatome, an die sie gebunden sind, einen spiroverknüpften Cyclopentan- oder Tetrahydropyranring darstellen,
R4 Carboxyl, Carboxymethyl, Carboxymethylmethyl, Methoxycarbonylmethyl, Methoxycarbonylmethylmethyl, Methoxycarbonyl, Aminocarbonyl oder Morpholin-4-yl-ethylenoxy-carbonyl bedeutet,
sowie die Salze dieser Verbindungen.

5. Verbindungen der Formel l nach Anspruch 1, in denen
Y O (Sauerstoff) oder NH bedeutet,
R1 Methoxy bedeutet,
R2 und R3 gemeinsam und unter Einschluß der beiden Kohlenstoffatome, an die sie gebunden sind, einen spiroverknüpften Cyclopentanring darstellen,
R4 Carboxyl, Carboxymethyl, Carboxymethylmethyl, Methoxycarbonylmethyl, Methoxycarbonylmethylmethyl, Methoxycarbonyl, Aminocarbonyl oder Morpholin-4-yl-ethylenoxy-carbonyl bedeutet,
sowie die Salze dieser Verbindungen.

6. Arzneimittel enthaltend eine oder mehrere Verbindungen nach Anspruch 1 zusammen mit üblichen pharmazeutischen Hilfs- und/oder Trägerstoffen.

7. Verbindungen nach Anspruch 1 zur Anwendung bei der Behandlung von Krankheiten.

8. Verwendung von Verbindungen nach Anspruch 1 zur Herstellung von Arzneimitteln für die Behandlung von Atemwegserkrankungen.

9. Verwendung von Verbindungen nach Anspruch 1 zur Herstellung von Arzneimitteln für die Behandlung von Dermatosen.

## Claims

1. Compounds of the formula I 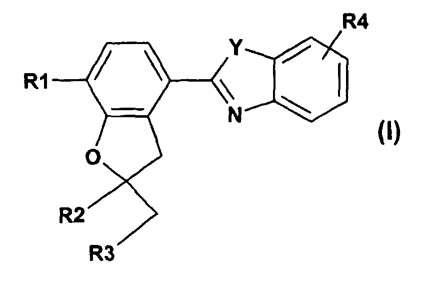 in which
Y is O (oxygen) or NH,
R1 is 1-4C-alkoxy, 3-7C-cycloalkoxy, 3-7C-cycloalkylmethoxy, benzyloxy or completely or predominantly fluorine-substituted 1-4C-alkoxy,
R2 is hydrogen or 1-4C-alkyl and
R3 is hydrogen or 1-4C-alkyl,
or in which
R2 and R3, together and including the two carbon atoms to which they are attached, are a spiro-linked 5-, 6- or 7-membered hydrocarbon ring which, optionally, is interrupted by an oxygen atom,
R4 is C(O)R5, C(O)R6, -CₙH₂ₙ-C(O)R5 or -CₙH₂ₙ-C(O)R6, where
R5 is hydroxyl, 1-7C-alkoxy, 3-7C-cycloalkoxy, 3-7C-cycloalkylmethoxy, H₂N-CₘH₂ₘ-O- or R51(R52)N-CₚH₂ₚ-O-, where
R51 is hydrogen, 1-7C-alkyl, 3-7C-cycloalkyl or 3-7C-cycloalkylmethyl and
R52 is 1-7C-alkyl, 3-7C-cycloalkyl or 3-7C-cycloalkylmethyl, or where
R51 and R52, together and including the nitrogen atom to which both are attached, are a 5-, 6- or 7-membered hydrocarbon ring which, optionally, is additionally interrupted by a group -N(R7)- or an oxygen atom,
R6 is N(R61)R62, where R61 and R62 independently of one another are hydrogen, 1-7C-alkyl, 3-7C-cycloalkyl or 3-7C-cycloalkylmethyl,
R7 is hydrogen or 1-4C-alkyl,
m is 2, 3 or 4,
n is 1,2, 3 or 4,
p is 1, 2, 3 or 4,
and the salts of these compounds.

2. Compounds of the formula I according to claim 1 in which
Y is O (oxygen) or NH,
R1 is 1-4C-alkoxy, 3-5C-cycloalkoxy, 3-5C-cycloalkylmethoxy, or completely or predominantly fluorine-substituted 1-2C-alkoxy,
R2 is 1-4C-alkyl and
R3 is hydrogen or 1-4C-alkyl,
or in which
R2 and R3, together and including the two carbon atoms to which they are attached, are a spiro-linked cyclopentane, cyclohexane, tetrahydrofuran or tetrahydropyran ring,
R4 is C(O)R5, C(O)R6, -CₙH₂ₙ-C(O)R5 or -CₙH₂ₙ-C(O)R6, where
R5 is hydroxyl, 1-7C-alkoxy, 3-7C-cycloalkoxy, 3-7C-cycloalkylmethoxy, H₂N-CₘH₂ₘ-O- or R51(R52)N-CₚH₂ₚ-O-, where
R51 is hydrogen or 1-4C-alkyl and
R52 is 1-4C-alkyl, or where
R51 and R52 together and including the nitrogen atom to which both are attached, are a 5-, 6- or 7-membered hydrocarbon ring which, optionally, is additionally interrupted by a group -N(R7)- or an oxygen atom,
R6 is N(R61 )R62, where R61 and R62 independently of one another are hydrogen or 1-4C-alkyl,
R7 is hydrogen or 1-4C-alkyl,
m is 2, 3 or 4,
n is 1 or 2,
p is 1, 2, 3 or 4,
and the salts of these compounds.

3. Compounds of the formula I according to claim 1 in which
Y is O (oxygen) or NH,
R1 is methoxy, ethoxy, cyclopropylmethoxy or completely or predominantly fluorine-substituted 1-2C-alkoxy,
R2 and R3, together and including the two carbon atoms to which they are attached, are a spiro-linked cyclopentane, cyclohexane, tetrahydrofuran or tetrahydropyran ring,
R4 is C(O)R5, C(O)R6, -CₙH₂ₙ-C(O)R5 or -CₙH₂ₙ-C(O)R6, where
R5 is hydroxyl, 1-4C-alkoxy or R51 (R52)N-CₚH₂ₚ-O-, where
R51 is hydrogen or 1-4C-alkyl and
R52 is 1-4C-alkyl, or where
R51 and R52, together and including the nitrogen atom to which both are attached, are a 6-membered hydrocarbon ring which, optionally, is interrupted by a group -N(R7)- or an oxygen atom,
R6 is N(R61)R62, where R61 and R62 independently of one another are hydrogen or 1-4C-alkyl,
R7 is hydrogen or 1-4C-alkyl,
n is 1 or 2,
p is 1, 2, 3 or 4,
and the salts of these compounds.

4. Compounds of the formula I according to claim 1 in which
Y is O (oxygen) or NH,
R1 is methoxy,
R2 and R3, together and including the two carbon atoms to which they are attached, are a spiro-linked cyclopentane or tetrahydropyran ring,
R4 is carboxyl, carboxymethyl, carboxymethylmethyl, methoxycarbonylmethyl, methoxycarbonylmethylmethyl, methoxycarbonyl, aminocarbonyl or morpholin-4-ylethyleneoxycarbonyl,
and the salts of these compounds.

5. Compounds of the formula I according to claim 1 in which
Y is O (oxygen) or NH,
R1 is methoxy,
R2 and R3, together and including the two carbon atoms to which they are attached, are a spiro-linked cyclopentane ring,
R4 is carboxyl, carboxymethyl, carboxymethylmethyl, methoxycarbonylmethyl, methoxycarbonylmethylmethyl, methoxycarbonyl, aminocarbonyl or morpholin-4-ylethyleneoxycarbonyl,
and the salts of these compounds.

6. Medicaments containing one or more compounds according to claim 1 together with the usual pharmaceutical auxiliaries and/or carrier materials.

7. Compounds according to claim 1 for the treatment of illnesses.

8. Use of compounds according to claim 1 for the production of medicaments for the treatment of airway disorders.

9. Use of compounds according to claim 1 for the production of medicaments for the treatment of dermatoses.

## Revendications

1. Composés de formule I 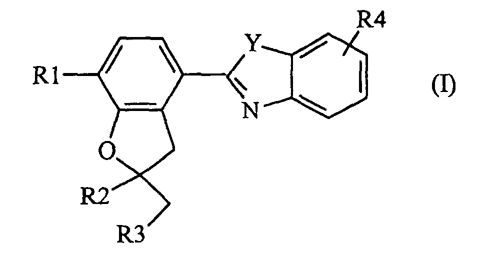 dans laquelle
Y représente un atome d'oxygène ou un groupe NH,
R1 représente un groupe alcoxy en C_{1-4,} cycloalcoxy en C_{3-7,} (cycloalkyle en C₃₋₇)méthoxy, benzyloxy ou alcoxy en C₁₋₄ totalement ou majoritairement fluoré,
R2 représente un atome d'hydrogène ou un groupe alkyle en C_{1-4,} et
R3 représente un atome d'hydrogène ou un groupe alkyle en C_{1-4,}
ou dans laquelle
R2 et R3 représentent, avec les deux atomes de carbone auxquels ils sont liés, un cycle hydrocarboné, lié en spiro, à 5, 6 ou 7 chaînons, comportant éventuellement un atome d'oxygène,
R4 représente un groupe C(O)R5, C(O)R6, -CₙH₂ₙ-C(O)R5 ou -CₙH₂ₙC(O)R6 où
R5 représente un groupe hydroxy, alcoxy en C_{1-7,} cycloalcoxy en C_{3-7,} (cycloalkyle en C₃₋₇)méthoxy, H₂N-CₘH₂ₘ-O- ou R51(R52)N-CₚH₂ₚ-O- où
R51 représente un atome d'hydrogène, un groupe alkyle en C_{1-7,} cycloalkyle en C₃₋₇ ou (cycloalkyle en C₃₋₇)méthyle, et
R52 représente un groupe alkyle en C_{1-7,} cycloalkyle en C₃₋₇ ou (cycloalkyle en C₃₋₇)méthyle, ou bien
R51 et R52 représentent conjointement, avec l'atome d'azote auquel ils sont liés, un cycle hydrocarboné à 5, 6 ou 7 chaînons, éventuellement interrompu par un chaînon supplémentaire qui est -N(R7)- ou un atome d'oxygène,
R6 représente un groupe N(R61)R62 où R61 et R62 représentent indépendamment l'un de l'autre un atome d'hydrogène, un groupe alkyle en C_{1-7,} cycloalkyle en C₃₋₇ ou (cycloalkyle en C₃₋₇)méthyle,
R7 représente un atome d'hydrogène ou un groupe alkyle en C_{1-4,}
m vaut 2, 3 ou 4,
n vaut 1, 2, 3 ou 4,
p vaut 1, 2, 3 ou 4,
ainsi que les sels de tels composés.

2. Composés de formule (I) selon la revendication 1, dans lesquels
Y représente un atome d'oxygène ou un groupe NH,
R1 représente un groupe alcoxy en C_{1-4,} cycloalcoxy en C₃₋₅, (cycloalkyle en C₃₋₅)méthoxy ou alcoxy en C₁₋₂ totalement ou majoritairement fluoré,
R2 représente un groupe alkyle en C_{1-4,} et
R3 représente un atome d'hydrogène ou un groupe alkyle en C_{1-4,}
ou dans lesquels
R2 et R3 représentent conjointement, avec les deux atomes de carbone auxquels ils sont liés, un groupe cyclopentane, cyclohexane, tétrahydrofurane ou tétrahydropyrane, lié en spiro,
R4 représente un groupe C(O)R5, C(O)R6, -CₙH₂ₙ-C(O)R5 ou - CₙH₂ₙC(O)R6 où
R5 représente un groupe hydroxy, alcoxy en C_{1-7,} cycloalcoxy en C_{3-7,} (cycloalkyle en C₃₋₇)-méthoxy, H₂N-CₘH₂ₘ-O- ou R51(R52)N-CₚH₂ₚ-O- où
R51 représente un atome d'hydrogène ou un groupe alkyle en C_{1-4,} et
R52 représente un groupe alkyle en C_{1-4,} ou bien
R51 et R52 représentent conjointement, avec l'atome d'azote auquel ils sont liés, un cycle hydrocarboné à 5, 6 ou 7 chaînons, éventuellement interrompu par un chaînon supplémentaire qui est -N(R7)- ou un atome d'oxygène,
R6 représente un groupe N(R61)R62 où R61 et R62 représentent indépendamment l'un de l'autre un atome d'hydrogène ou un groupe alkyle ne C_{1-4,}
R7 représente un atome d'hydrogène ou un groupe alkyle en C_{1-4,}
m vaut 2, 3 ou 4,
n vaut 1 ou 2,
p vaut 1, 2, 3 ou 4,
ainsi que les sels de ces composés.

3. Composés de formule (I) selon la revendication 1, dans lesquels
Y représente un atome d'oxygène ou un groupe NH,
R1 représente un groupe méthoxy, éthoxy, cyclopropylméthoxy ou alcoxy en C₁₋₂ totalement ou majoritairement fluoré,
R2 et R3 représentent conjointement, avec les deux atomes de carbone auxquels ils sont liés, un groupe cyclopentane, cyclohexane, tétrahydrofurane ou tétrahydropyrane, lié en spiro,
R4 représente un groupe C(O)R5, C(O)R6, -CₙH₂ₙ-C(O)R5 ou -CₙH₂ₙC(O)R6, où
R5 représente un groupe hydroxy, alcoxy en C₁₋₄ ou R51(R52)N-CₚH₂ₚ-O- où
R51 représente un atome d'hydrogène ou un groupe alkyle en C_{1-4,}
R52 représente un groupe alkyle en C_{1-4,} ou bien
R51 et R52 représentent conjointement, avec l'atome d'azote auquel ils sont liés, un cycle hydrocarboné à 6 chaînons, éventuellement interrompu par un groupe -N(R7)- ou un atome d'oxygène,
R6 représente un groupe N(R61)R62 où R61 et R62 représentent indépendamment l'un de l'autre un atome d'hydrogène ou un groupe alkyle en C_{1-4,}
R7 représente un atome d'hydrogène ou un groupe alkyle en C_{1-4,}
n vaut 1 ou 2,
p vaut 1, 2, 3 ou 4,
ainsi que les sels de tels composés.

4. Composés de formule (I) selon la revendication 1, dans lesquels
Y représente un atome d'oxygène ou un groupe NH,
R1 représente un groupe méthoxy,
R2 et R3 représentent conjointement, avec les deux atomes de carbone auxquels ils sont liés, un groupe cyclopentane ou tétrahydropyrane, lié en spiro,
R4 représente un groupe carboxyle, carboxyméthyle, carboxyméthylméthyle, méthoxycarbonylméthyle, méthoxycarbonylméthylméthyle, méthoxycarbonyle, aminocarbonyle ou morpholin-4-yléthylèneoxycarbonyle,
et les sels de tels composés.

5. Composés de formule (I) selon la revendication 1, dans lesquels
Y représente un atome d'oxygène ou un groupe NH,
R1 représente un groupe méthoxy,
R2 et R3 représentent conjointement, avec les deux atomes de carbone auxquels ils sont liés, un groupe cyclopentane lié en spiro,
R4 représente un groupe carboxyle, carboxyméthyle, carboxyméthylméthyle, méthoxycarbonylméthyle, méthoxycarbonyl-méthylméthyle, méthoxycarbonyle, aminocarbonyle ou morpholin-4-yléthylèneoxycarbonyle,
et les sels de tels composés.

6. Médicament contenant un ou plusieurs composés selon la revendication 1 avec des adjuvants et/ou excipients pharmaceutiques usuels.

7. Composés selon la revendication 1 pour une utilisation pour le traitement de maladies.

8. Utilisation de composés selon la revendication 1, pour la fabrication de médicaments destinés au traitement de maladies respiratoires.

9. Utilisation de composés selon la revendication 1, pour la fabrication de médicaments destinés au traitement de dermatoses.
